# EUROPÄISCHE PATENTSCHRIFT

(11) **EP 1 009 787 B1**
(45) Veröffentlichungstag und Bekanntmachung des Hinweises auf die Patenterteilung: **08.09.2004**
(21) Anmeldenummer: 99931238.2
(22) Anmeldetag: 02.07.1999
(51) Int. Cl.: C11D 1/29, B01F 7/18

(54) **VERFAHREN ZUR HERSTELLUNG VON WÄSSRIGEN LÖSUNGEN VON ALKYLETHERSULFATEN**
METHOD FOR PRODUCING AQUEOUS SOLUTIONS OF ALKYL ETHER SULFATES
PROCEDE POUR LA PREPARATION DE SOLUTIONS AQUEUSES DE SULFATES D'ALKYLETHER

(30) Priorität: 02.07.1998 DE 19829646
(43) Veröffentlichungstag der Anmeldung: 21.06.2000
(73) Patentinhaber: Wella Aktiengesellschaft, 64274 Darmstadt (DE)
(72) Erfinder: HUFF, Rainer, D-36166 Hünfeld (DE); FRAUENRIEDER, Gerd, D-36151 Burghaun 3/Rothenkirchen (DE); BIEBER, Bernhard, D-36088 Hünfeld (DE)
(86) Internationale Anmeldenummer: PCT/EP1999/004588
(87) Internationale Veröffentlichungsnummer: WO 2000/001792

(56) Entgegenhaltungen:
- EP-A- 0 760 254
- DE-A- 19 500 799
- GB-A- 1 437 089
- US-A- 3 786 003
- ZIOLKOWSKY B.: "Mischer zur Herstellung von Badepräparaten und Emulsionen" SEIFEN-ÖLE-FETTE-WACHSE, Bd. 112, Nr. 9, 1986, Seiten 311-314, XP002119512 in der Anmeldung erwähnt
- ZIOLKOWSKY B.: "Mischer zur Herstellung von Badepräparaten und Emulsionen" SEIFEN-ÖLE-FETTE-WACHSE, Bd. 112, Nr. 15, 1986, Seiten 532-536, XP002119513 in der Anmeldung erwähnt

## Beschreibung

Gegenstand der Erfindung ist ein Verfahren zur Herstellung von wäßrigen Lösungen von Alkylethersulfaten.

Die am häufigsten eingesetzten Tenside zur Herstellung von tensidhaltigen Reinigungszusammensetzungen sind Alkylethersulfate, insbesondere Laurylethersulfat. Alkylethersulfat wird überwiegend als Natrium- oder Ammoniumsalz mit einem Ethoxylierungsgrad von zwei bis drei eingesetzt und üblicherweise in hochkonzentrierter, pastöser Form als 70%ige Ware angeboten, die vor der Verwendung verdünnt werden muß. Der Vorteil der hochkonzentrierten Form liegt vor allem in Kostenersparnissen beim Transport, bei Platzersparnissen bei der Lagerung sowie aus mikrobiologischer Sicht in der Keimstabilität. Bei der Herstellung von Reinigungszusammensetzungen nimmt die Verdünnung der hochkonzentrierten, pastösen, 70%igen Alkylethersulfate eine besondere Stellung ein. Die Verdünnung gestaltet sich wegen des Viskositätsverhaltens schwierig. Im Konzentrationsbereich von 65 bis 75% liegt ein Viskositätsminimum vor. Im Konzentrationsbereich von 30 bis 60% liegen die Alkylethersulfate als äußerst hochviskose, steife Gele und erst bei Konzentrationen unterhalb von 30% (bezogen auf wäßrige Verdünnung) in niedrigviskoser, gut verarbeitbarer Form vor. Selbst bei Verwendung von Hochleistungsmischern bilden sich beim Eintrag von konzentriertem Alkylethersulfat in Wasser Ausgeelierungen oder gelartige Knoten, die eine sofortige und vollständige Lösung verhindern und die sich, wenn überhaupt, erst nachträglich langsam lösen. Dies hat beispielsweise das unerwünschte Nachverdicken der Fertigprodukte zur Folge, das heißt die endgültige viskosität und Konsistenz stellt sich erst nach einer gewissen Lagerungszeit ein. Die Problematik des Verdünnens von hochkonzentrierten Alkylethersulfaten wird beispielsweise' beschrieben in Andreas Domsch, Die kosmetischen Präparate, Band II, 4. Auflage, Seiten 255 bis 256, in dem Artikel in Parfümerie und Kosmetik, 75. Jahrgang, Nr. 1/94, Seiten 26 bis 35 sowie in dem Artikel in Seifen-Öle-Fette-Wachse, 112. Jahrgang, Nr. 9/86, Seiten 311 bis 314.

Übliche Maßnahmen zur Umgehung der viskositätsbedingten Schwierigkeiten bestehen darin, daß das Produkt über längere Zeit im Kreis über einen Homogenisator geführt wird, um eine möglichst vollständige Lösung zu erreichen, was jedoch sehr zeitintensiv ist. Außerdem wird üblicherweise dem Lösungsmittel ein Elektrolyt wie Natriumchlorid zugesetzt. Natriumchlorid bewirkt in dem Konzentrationsbereich, in dem Alkylethersulfate normalerweise als steife Gele vorliegen (30-60%), eine deutliche Viskositätserniedrigung und damit eine verbesserte Verarbeitbarkeit und schnellere Lösung. Bei den daraus resultierenden verdünnten Alkylethersulfatlösungen hat Natriumchlorid allerdings die entgegengesetzte, nämlich eine viskositätserhöhende Wirkung. Dies hat den Nachteil, daß für die derart hergestellten, viskosen Lösungen ein erhöhter Zeitbedarf für das nachfolgende Entgasen anzusetzen ist und daß die Weiterbearbeitung, beispielsweise das nachfolgende Einbringen von zusätzlichen Wirk- und Hilfsstoffen schwieriger und zeitintensiver ist. Außerdem kann auch durch diese Maßnahmen ein Nachverdicken nicht ausgeschlossen werden.

Es bestand somit die Aufgabe, ein verbessertes Verfahren zur Herstellung von wäßrigen Lösungen von Alkylethersulfaten zur Verfügung zu stellen, welches zeit- und kostengünstig ist und gleichzeitig die gewünschten Eigenschaften der hergestellten Produkte nicht wesentlich beeinträchtigt oder sogar noch verbessert.

Gelöst wird die Aufgabe durch ein Verfahren zur Herstellung von wäßrigen Lösungen von Alkylethersulfaten wobei
(A) in einem Behälter eine vorzugsweise elektrolytfreie wäßrige Phase vorgelegt wird, wobei an den Behälter ein Homogenisator vom Rotor/Stator-Typ angeschlossen ist und der Homogenisator einen zusätzlichen Anschluß aufweist, durch den eine einzuhomogenisierende Substanz direkt auf den Rotor aufgegeben werden kann und die Substanz erst in den Zahnkränzen des Homogenisators mit der externen wäßrigen Phase in Berührung kommt
(B) der Homogenisator gestartet wird und anschließend
(C) hochkonzentriertes Alkylethersulfat über den zusätzlichen Anschluß des Homogenisators zugeführt wird.

Der Begriff 'wäßrige Phase' umfaßt Wasser sowie Gemische von Wasser mit wasserlöslichen Lösungsmitteln wie niederen Alkoholen, z.B. Ethanol oder Isopropanol oder Polyolen wie Ethylenglykol, Diethylenglykol, Butylenglykol oder Glycerin, vorzugsweise jedoch Wasser.

Der Behälter braucht kein zusätzliches Rührwerk zu besitzen. Es kann sich also auch um einen einfachen Lagertank handeln, an den der Homogenisator angeschlossen ist.

Das Verfahren kann auch in einer kontinuierlichen Variante durchgeführt werden (Durchflußverfahren).
Bei dem kontinuierlichen Verfahren wird
(A) eine vorzugsweise elektrolytfreie wäßrige Phase kontinuierlich einem Homogenisator zugeführt, wobei der Homogenisator vom Rotor/Stator-Typ ist und der Homogenisator einen zusätzlichen Anschluß aufweist, durch den eine einzuhomogenisierende Substanz direkt auf den Rotor aufgegeben werden kann und die Substanz erst in den Zahnkränzen des Homogenisators mit der externen Phase in Berührung kommt,
(B) der Homogenisator wird gestartet und anschließend
(C) wird hochkonzentriertes Alkylethersulfat über den zusätzlichen Anschluß des Homogenisators kontinuierlich zugeführt.

Geeignete Alkylethersulfate weisen eine Alkylgruppe mit 8 bis 22, bevorzugt von 10 bis 16 Kohlenstoffatomen und einen Ethoxylierungsgrad von 1 bis 20, bevorzugt von 1 bis 4 auf. Besonders bevorzugt sind Laurylethersulfate. Geeignete Gegenionen sind Alkali- oder Erdalkaliionen beispielsweise Natriumionen, Magnesiumionen oder auch Ammoniumionen. Geeignete Alkylethersulfate sind beispielsweise die im 'International Cosmetic Ingredient Dictionary and Handbook', 7. Auflage, Band 2 im Abschnitt 'Alkyl Ether Sulfates' aufgeführten Tenside.

Ein wesentlicher Bestandteil des Verfahrens ist der einzusetzende Homogenisator. Hierbei handelt es sich um einen Homogenisator vom Rotor/Stator-Typ, der einen zusätzlichen Anschluß in einer ganz bestimmten Position aufweist. Herkömmliche Rotor/Stator-Homogenisatoren werden beispielsweise beschrieben in Andreas Domsch, 'Die kosmetischen Präparate', Band III, 4. Auflage, Seite 305 bis 307 sowie in dem Artikel in Seifen-Öle-Fette-Wachse, 112. Jahrgang, 1986, Seiten 532 bis 536. Das Rotor-Stator-Prinzip ist das am häufigsten angewendete Homogenisierverfahren bei der Herstellung kosmetischer Präparate. Hierbei wird das zu homogenisierende Gut mittels eines sich drehenden Teils, dem Rotor, durch einen feststehenden Teil, den Stator hindurchbewegt, wobei zwischen dem Rotor und dem Stator nur ein äußerst geringer Spalt besteht. Die Homogenisierwirkung beruht auf den im Scherspalt auftretenden Turbulenzen. Bei Verwendung von herkömmlichen Homogenisatoren ohne einen zusätzlichen Anschluß ist die einzuhomogenisierende Substanz bereits in Kontakt mit der wäßrigen Phase, liegt also als vormischung oder Voremulsion vor. Mit diesen herkömmlichen Homogenisatoren sind Lösungen nur nach den herkömmlichen Verfahren, nicht jedoch nach dem erfindungsgemäßen Verfahren herstellbar. Nach dem erfindungsgemäßen Verfahren ist ein Kontakt des Alkylethersulfats mit der äußeren, wäßrigen Phase vor dem eigentlichen Homogenisiervorgang zu vermeiden.

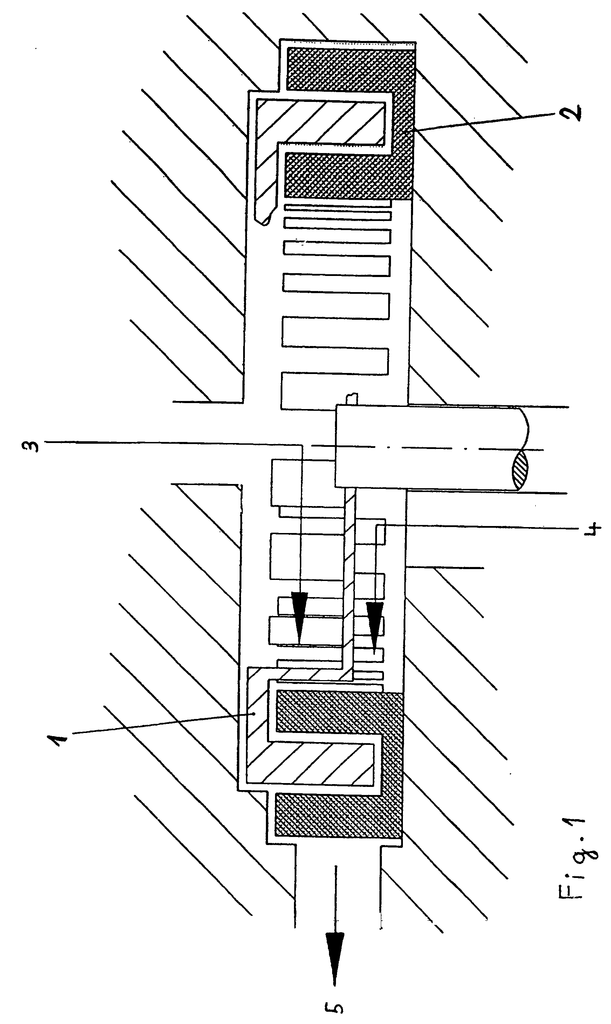

Ein geeigneter Homogenisator mit Rotor (1) und Stator (2) ist in Figur 1 dargestellt. Die vorgelegte Phase (3) kommt erst in den Zahnkränzen mit der einzuhomogenisierenden, zweiten Phase (4) in Berührung. Das Fertigprodukt (5) tritt in Form einer feinen Dispersion aus.

Ein weiterer Gegenstand der Erfindung ist die Verwendung eines Homogenisators vom Rotor/Stator-Typ, welcher einen zusätzlichen Anschluß aufweist, durch den eine einzuhomogenisierende Substanz direkt auf den Rotor aufgegeben werden kann und die Substanz erst in den Zahnkränzen des Homogenisators mit der externen, wäßrigen Phase in Berührung kommt, zum Lösen von Alkylethersulfaten in Wasser oder zur Herstellung von Alkylethersulfate enthaltenden Zusammensetzungen.

Es wurden Versuche zur Lösung von Alkylethersulfat in Wasser durchgeführt, bei denen konzentriertes, 70%iges Laurylethersulfat über einen zusätzlichen Anschluß in den Zufluß zum Homogenisator eingespeist wurde, wobei der zusätzliche Anschluß unmittelbar vor der Rotor/Stator-Einheit positioniert wurde. Diese Versuche führten nicht zu einem befriedigenden Ergebnis, da hier immer noch ein, wenn auch nur sehr kurzzeitiger, Kontakt zwischen einzuhomogenisierender Substanz und wäßriger Phase vor dem eigentlichen Homogenisiervorgang vorliegt. Vermutlich kommt es zu Ausgeelierungen, Klumpenbildungen oder Bildungen von gelartigen Knoten, die zumindest eine nicht sofortige vollständige Lösung oder eine längere Ansatzzeit bewirken und im Extremfall sogar zum Blockieren des Rotors führen können. Erfindungsgemäß muß der Homogenisator daher über einen zusätzlichen Anschluß verfügen, der so positioniert ist, daß die einzuhomogenisierende Substanz direkt in die Verzahnung der Rotor/Stator-Einheit gelangt, ohne zuvor mit der übrigen äußeren Phase in Kontakt gekommen zu sein. Im Prinzip kann jeder handelsübliche Homogenisator verwendet werden, sofern er konstruktiv in der beschriebenen Weise verändert ist. Ein besonders geeigneter Homogenisator wird beispielsweise von der Firma Berents unter der Bezeichnung Becomix Duo-Homogenisator DH 2000 angeboten.

Das Verhältnis der dem Homogenisator zufließenden Teilströme von vorgelegter Phase einerseits und Alkylethersulfatkonzentrat andererseits werden idealerweise so eingestellt, daß das Alkylethersulfat nicht in einen den Zahnkränzen der Rotor/Statoreinheit vorgelagerten Bereich transportiert wird, wo es vor Homogenisierung mit der vorgelegten Phase in Kontakt kommen kann. Die Einstellung des Alkylethersulfat zuführenden Teilstroms kann durch Anlegen eines Druckunterschieds erfolgen. Vorzugsweise wird ein leichter Unterdruck von beispielsweise 0,2 bis 0,8 bar, vorzugsweise 0,3 bis 0,6 bar angelegt. Die Einstellung des die vorgelegte Phase zuführenden Teilstroms kann durch Einstellung der Umfangsgeschwindigkeit des Rotors erfolgen. Bei typischen, handelsüblichen Rotoren beträgt die Umfangsgeschwindigkeit beispielsweise 20 bis 40 m/s, vorzugsweise 25 bis 30 m/s.

In einer besonderen Ausführungsform des Verfahrens wird die primär erhaltene, aus dem Homogenisator herausströmende Alkylethersulfat enthaltende Zusammensetzung dem Voratsbehälter im Kreislauf wieder zurückgeführt. Vorzugsweise erfolgt die Rückführung zur Vermeidung von übermäßiger Schaumbildung unterhalb des Flüssigkeitsspiegels des Vorratsbehälters. Ein weiterer Gegenstand der Erfindung ist daher eine Vorrichtung zur Durchführung dieser besonderen Ausführungsform, bestehend aus mindestens
(a) einem Vorratsbehälter, an den angeschlossen ist
(b) ein Homogenisator vom Rotor/Stator-Typ welcher einen zusätzlichen Anschluß aufweist, durch den eine einzuhomogenisierende Substanz direkt auf den Rotor aufgegeben werden kann und die Substanz erst in den Zahnkränzen des Homogenisators mit der externen, wäßrigen Phase in Berührung kommt und
(c) einer rohrartigen Verbindung zur Rückführung der homogenisierten Mischung in den Vorratsbehälter, wobei die Rückführung vorzugsweise unterhalb des Flüssigkeitsspiegels des Vorratsbehälters erfolgt.

Soll es sich bei der fertigen Alkylethersulfatlösung um eine viskose Zusammensetzung handeln, so wird die gewünschte Endviskosität vorzugsweise zum Abschluß des Herstellungsverfahrens durch Zugabe eines Elektrolyten wie Natriumchlorid oder eines anderen, üblichen viskositätssteigernden Stoffes eingestellt. Hierzu können Verdicker verwendet werden wie beispielsweise Guar Gums, Silikate, Methylcellulose, Hydroxyethylcellulose oder Carboxyvinylpolymere.

Das erfindungsgemäße Verfahren eignet sich zur Herstellung von Reinigungsmittelzusammensetzungen, beispielsweise flüssige oder gelförmige Körperreinigungsmittel wie Shampoos oder Duschgele aber auch für Haushaltsreiniger. Hierzu wird zunächst Alkylethersulfat nach dem oben beschriebenen Verfahren in vorzugsweise elektrolytfreiem Wasser gelöst. Anschließend werden die weiteren Wirk- und Zusatzstoffe eingerührt. Als Wirkund Zusatzstoffe kommen beispielsweise Stoffe in Frage, wie sie in kosmetischen Formulierungen üblich sind wie z.B. Pflegestoffe wie quaternisierte Alkylamine, kationische Polymere natürlichen oder synthetischen Ursprungs, Proteine und ihre Derivate, wie z.B.

Kollagen-, Keratin-, Seidenprotein- und Weizenproteinhydrolysate. Desweiteren können eingesetzt werden: Parfümöle, Farbstoffe, Trübungsmittel wie z.B. Glycoldistearat; weitere Verdicker wie Guar Gums, Silikate, Methylcellulose, Hydroxyethylcellulose, Carboxyvinylpolymere; weitere haarkonditionierende Mittel wie synthetische oder natürliche Phospholipide oder quaternäre Derivate der Stärke oder Cellulose; Lösungsvermittler wie kurzkettige Alkohole, z.B. Ethanol, n- und iso-Propanol oder Glykole wie Butylen- oder Propylenglykol; Aminosäuren wie z.B. Histidin, Glycin, Alanin, Threonin, Arginin, Cystein und deren Derivate wie z.B. Fettsäurekondensationsprodukte oder quaternäre Produkte; weitere Wirkstoffe wie Pflanzenextrakte, Vitamine, Allantoin, Chitosan, Konservierungsmittel u.v.m..

Da die erfindungsgemäß primär hergestellte Alkylethersulfatlösung äußerst niedrigviskos (wasserdunn) ist, lassen sich die weiteren Wirk- und Zusatzstoffe wesentlich einfacher und schneller einarbeiten als nach herkömmlichen Verfahren, wodurch die Ansatzzeiten deutlich reduziert werden. Insbesondere sind keine längeren Standzeiten zum Entgasen erforderlich. Soll es sich bei den Endprodukten um viskose Zusammensetzungen handeln, so wird die gewünschte Endviskosität vorzugsweise zum Abschluß des Herstellungsverfahrens durch Zugabe eines Elektrolyten wie Natriumchlorid oder eines anderen, üblichen viskositätssteigernden Stoffes eingestellt. Hierzu können Verdicker verwendet werden wie beispielsweise Guar Gums, Silikate, Methylcellulose, Hydroxyethylcellulose oder Carboxyvinylpolymere.

Nach herkömmlichen Verfahren hergestellte Zusammensetzungen zeigen häufig den Effekt des Nachverdickens, das heißt die engültige Viskosität und Konsistenz stellt sich erst nach einer gewissen Lagerungszeit ein. Dies ist möglicherweise darauf zurückzuführen, daß noch mikroskopisch kleine gelartige Alkylethersulfatteilchen vorliegen, die sich erst nachträglich lösen, wodurch sich die Viskosität verändert. Nach dem erfindungsgemäßen Verfahren hergestellte Zusammensetzungen weisen diesen Nachteil nicht auf, sondern besitzen bereits direkt nach Abschluß des Herstellverfahrens ihre endgültige Viskosität.

Es hat sich gezeigt, daß bei erfindungsgemäß hergestellten Laurylethersulfatlösungen bei gleicher Konzentration an Laurylethersulfat eine deutlich geringere Menge an Natriumchlorid benötigt wird um eine bestimmte Endviskosität einzustellen, als bei nicht erfindungsgemäß hergestellten Lösungen. Die höhere Wirksamkeit des Natriumchlorids ist vermutlich auf eine optimalere Verteilung des Laurylethersulfats zurückzuführen.

### Beispiele

### Beispiel 1: Lösen von Laurylethersulfat in Wasser

107,2 kg Wasser wurden kalt in einem Kessel vorgelegt, an den ein Homogenisator vom Typ Becomix Duo-Homogenisator DH 2000 (Berents GmbH & Co. KG) angeschlossen war. Der Homogenisator wurde gestartet und es wurden 52 kg 70%iges Laurylethersulfat über den zusätzlichen Anschluß des Homogenisators in das Wasser eingetragen und auf diese Weise gelöst. Die entstandene Lösung ist sehr niedrigviskos, gewissermaßen wasserdünn.

### Beispiel 2: Herstellung eines Shampoos

105,4 kg Wasser wurden kalt (15 °C) in einem Rührkessel vorgelegt, an den ein Homogenisator vom Typ Becomix Duo-Homogenisator DH 2000 (Berents GmbH & Co. KG) angeschlossen war. Der Homogenisator wurde gestartet und es wurden 30,6 kg 70%iges Laurylethersulfat über den zusätzlichen Anschluß des Homogenisators in das Wasser eingetragen und auf diese Weise gelöst. Es wurden 2 kg einer Schmelze des Trübungsmittels Polyethylenglykol-(3)-distearat (75°C) über den zusätzlichen Anschluß des Homogenisators in die wäßrige Tensidlösung eingetragen. Anschließend wurden die Zusatzbestandteile wie Haarpflegestoffe, Parfüm und Konservierungsmittel von oben zugegeben und eingerührt. Die Einstellung der Viskosität erfolgte mit Natriumchlorid, die Einstellung des pH-Wertes erfolgte mit Zitronensäure. Zugabe von 0,6 kg Natriumchlorid ergab eine Viskosität von 3590 mPas bei 25°C.

Es wurde eine sehr homogene Masse mit gut entwickeltem Perlglanz erhalten. Bei einem Stabilitätstest in einer Zentrifuge (3000 U/min, 5 Minuten) wurde keine Auftrennung der Masse festgestellt.

Bei auf herkömmlichem Weg hergestellten, trüben Shampooformulierungen beobachtet man dagegen früher oder später (je nach Bedingungen oder Formulierung) eine Neigung zum Auftrennen der Phasen.

## Patentansprüche

1. Verfahren zur Herstellung von wäßrigen Lösungen von Alkylethersulfaten wobei
(A) in einem Behälter eine vorzugsweise elektrolytfreie wäßrige Phase vorgelegt wird, wobei an den Behälter ein Homogenisator vom Rotor/Stator-Typ angeschlossen ist und der Homogenisator einen zusätzlichen Anschluß aufweist, durch den eine einzuhomogenisierende Substanz direkt auf den Rotor aufgegeben werden kann und die Substanz erst in den Zahnkränzen des Homogenisators mit der externen, wäßrigen Phase in Berührung kommt
(B) der Homogenisator gestartet wird und anschließend
(C) hochkonzentriertes Alkylethersulfat über den zusätzlichen Anschluß des Homogenisators zugeführt wird.

2. Kontinuierliches Verfahren zur Herstellung von wäßrigen Lösungen von Alkylethersulfaten wobei
(A) eine vorzugsweise elektrolytfreie wäßrige Phase kontinuierlich einem Homogenisator zugeführt wird, wobei der Homogenisator vom Rotor/Stator-Typ ist und der Homogenisator einen zusätzlichen Anschluß aufweist, durch den eine einzuhomogenisierende Substanz direkt auf den Rotor aufgegeben werden kann und die Substanz erst in den Zahnkränzen des Homogenisators mit der externen, wäßrigen Phase in Berührung kommt
(B) der Homogenisator gestartet wird und anschließend
(C) hochkonzentriertes Alkylethersulfat über den zusätzlichen Anschluß des Homogenisators kontinuierlich zugeführt wird.

3. Verfahren nach Anspruch 1 oder 2, **dadurch gekennzeichnet, daß** zum Schluß durch Zugabe von Elektrolyten oder anderen viskositätserhöhenden Stoffen die Endviskosität der Lösung eingestellt wird.

4. Verfahren zur Herstellung einer Reinigungsmittelzusammensetzung, wobei zunächst eine wäßrige Alkylethersulfatlösung nach Anspruch 1 oder 2 hergestellt wird und anschließend die übrigen Wirk- und Zusatzstoffe eingerührt werden.

5. Verfahren nach Anspruch 4, **dadurch gekennzeichnet, daß** zum Schluß durch Zugabe von Elektrolyten oder anderen viskositätserhöhenden Stoffen die Endviskosität der Reinigungsmittelzusammensetzung eingestellt wird.

6. Verfahren nach Anspruch 4 oder 5, **dadurch gekennzeichnet, daß** es sich um ein Körperreinigungsmittel handelt.

7. Verfahren nach einem der vorstehenden Ansprüche, **dadurch gekennzeichnet, daß** es sich bei dem Alkylethersulfat um Laurylethersulfat handelt.

8. Verfahren nach Anspruch 1, **dadurch gekennzeichnet, daß** die Alkylethersulfat enthaltende Mischung oder Lösung in den die vorgelegte Phase enthaltenden Vorratsbehälter zurückgeführt wird.

9. Verfahren nach Anspruch 8, **dadurch gekennzeichnet, daß** die Rückführung der Alkylethersulfat enthaltenden Mischung oder Lösung unterhalb des Flüssigkeitsspiegels des Vorratsbehälters erfolgt.

10. Verfahren nach einem der vorgenannten Ansprüche, **dadurch gekennzeichnet, daß** das Verhältnis des Alkylethersulfat zuführenden Teilstroms zu dem die vorgelegte Phase zuführenden Teilstrom so gewählt ist, daß das Alkylethersulfat nicht in einen den Zahnkränzen der Rotor/Statoreinheit vorgelagerten Bereich transportiert wird, wo es vor Homogenisierung mit der vorgelegten Phase in Kontakt kommen kann.

11. Verfahren nach Anspruch 10, **dadurch gekennzeichnet, daß** der Alkylethersulfat zuführende Teilstrom durch Anlegen eines Druckunterschieds eingestellt wird und der die vorgelegte Phase zuführende Teilstrom durch Einstellung der Umfangsgeschwindigkeit des Rotors eingestellt wird.

12. Verwendung eines Homogenisators vom Rotor/Stator-Typ welcher einen zusätzlichen Anschluß aufweist, durch den eine einzuhomogenisierende Substanz direkt auf den Rotor aufgegeben werden kann und die Substanz erst in den Zahnkränzen des Homogenisators mit der externen, wäßrigen Phase in Berührung kommt, zum Lösen von Alkylethersulfaten in Wasser oder zur Herstellung von Alkylethersulfate enthaltenden Zusammensetzungen.

## Claims

1. Process for the preparation of aqueous solutions of alkyl ether sulphates where
(A) a preferably electrolyte-free aqueous phase is initially introduced into a container, where a homogenizer of the rotor/stator type is connected to the container, and the homogenizer has an additional connection through which a substance to be incorporated by homogenization can be placed directly on the rotor, and the substance comes into contact with the external, aqueous phase only in the toothed-wheels of the homogenizer
(B) the homogenizer is started and then
(C) highly concentrated alkyl ether sulphate is introduced by the additional homogenizer connection.

2. Continuous process for the preparation of aqueous solutions of alkyl ether sulphates where
(A) a preferably electrolyte-free aqueous phase is introduced continuously into a homogenizer, where the homogenizer is of the rotor/stator type and the homogenizer has an additional connection through which a substance to be incorporated by homogenization can be placed directly onto the rotor, and the substances comes into contact with the external, aqueous phase only in the toothed-wheels of the homogenizer
(B) the homogenizer is started and then
(C) highly concentrated alkyl ether sulphate is introduced continuously by the additional homogenizer connection.

3. Process according to Claim 1 or 2, **characterized in that**, finally, the final viscosity of the solution is adjusted by adding electrolytes or other viscosity-increasing substances.

4. Process for the preparation of a cleaning composition, where firstly an aqueous alkyl ether sulphate solution is prepared according to Claim 1 or 2, and then the other active ingredients and additives are stirred in.

5. Process according to Claim 4, **characterized in that**, finally, the final viscosity of the cleaning composition is adjusted by adding electrolytes or other viscosity-increasing substances.

6. Process according to Claim 4 or 5, **characterized in that** it is a body-cleansing product.

7. Process according to one of the preceding claims, **characterized in that** the alkyl ether sulphate is lauryl ether sulphate.

8. Process according to Claim 1, **characterized in that** the mixture or solution containing alkyl ether sulphate is returned to the storage container containing the initially introduced phase.

9. Process according to Claim 8, **characterized in that** the mixture or solution containing alkyl ether sulphate is returned below the level of liquid in the storage container.

10. Process according to one of the preceding claims, **characterized in that** the ratio of the partial stream introducing alkyl ether sulphate to the partial stream introducing, the initially introduced phase is chosen such that the alkyl ether sulphate is not transported to a region upstream of the toothed-wheels of the rotor/stator unit where it can come into contact with the initially introduced phase prior to homogenization.

11. Process according to Claim 10, **characterized in that** the partial stream introducing alkyl ether sulphate is adjusted by applying a pressure difference, and the partial stream introducing the initially introduced phase is adjusted by adjusting the peripheral speed of the rotor.

12. Use of a homogenizer of the rotor/stator type which has additional connection through which a substance to be incorporated by homogenization can be placed directly onto the rotor, and the substance comes into contact with the external, aqueous phase only in the toothed-wheels of the homogenizer, for dissolving alkyl ether phosphates in water or for producing compositions comprising alkyl ether sulphates.

## Revendications

1. Procédé pour la préparation de solutions aqueuses d'alkyléthersulfates, dans lequel
(A) on dispose au préalable dans un récipient une phase aqueuse, de préférence exempte d'électrolytes, un homogénéisateur du type rotor/stator étant raccordé au récipient et l'homogénéisateur comportant un raccord supplémentaire par lequel une substance à incorporer de façon homogène peut être amenée directement sur le rotor et la substance n'entrant en contact avec la phase aqueuse externe que dans les couronnes dentées de l'homogénéisateur,
(B) on met en marche l'homogénéisateur et ensuite
(C) on envoie à l'homogénéisateur, par le raccord supplémentaire, un alkyléthersulfate hautement concentré.

2. Procédé continu pour la préparation de solutions aqueuses d'alkyléthersulfates, dans lequel
(A) on envoie en continu à un homogénéisateur une phase aqueuse, de préférence exempte d'électrolytes, l'homogénéisateur étant du type rotor/stator et l'homogénéisateur comportant un raccord supplémentaire par lequel une substance à incorporer de façon homogène peut être amenée directement sur le rotor et la substance n'entrant en contact avec la phase aqueuse externe que dans les couronnes dentées de l'homogénéisateur,
(B) on met en marche l'homogénéisateur et ensuite
(C) on envoie en continu à l'homogénéisateur, par le raccord supplémentaire, un alkyléthersulfate hautement concentré.

3. Procédé selon la revendication 1 ou 2, **caractérisé en ce qu'**à la fin on ajuste la viscosité finale de la solution par addition d'électrolytes ou d'autres substances augmentant la viscosité.

4. Procédé pour la préparation d'une composition de produit de nettoyage, dans lequel on prépare d'abord une solution aqueuse d'alkyléthersulfate selon la revendication 1 ou 2 et on délaie ensuite les autres actifs et additifs.

5. Procédé selon la revendication 4, **caractérisé en ce qu'**à la fin on ajuste la viscosité finale de la composition de produit de nettoyage par addition d'électrolytes ou d'autres substances augmentant la viscosité.

6. Procédé selon la revendication 4 ou 5, **caractérisé en ce qu'**il s'agit d'un produit de nettoyage corporel.

7. Procédé selon l'une quelconque des revendications précédentes, **caractérisé en ce que** l'alkyléthersulfate est le lauryléthersulfate.

8. Procédé selon la revendication 1, **caractérisé en ce que** la solution ou le mélange contenant l'alkyléthersulfate est renvoyé dans le réservoir contenant la phase disposée au préalable.

9. Procédé selon la revendication 8, caractérisé en ce le renvoi de la solution ou du mélange contenant l'alkyléthersulfate s'effectue au-dessous du niveau du liquide du réservoir.

10. Procédé selon l'une quelconque des revendications précédentes, **caractérisé en ce que** le rapport du courant partiel amenant l'alkyléthersulfate au courant partiel amenant la phase disposée au préalable est choisi de manière que l'alkyléthersulfate ne soit pas transporté dans une zone placée avant les couronnes dentées de l'unité de rotor/stator, où elle peut entrer en contact avant l'homogénéisation avec la phase disposée au préalable.

11. Procédé selon la revendication 10, **caractérisé en ce que** le courant partiel amenant l'alkyléthersulfate est réglé par application d'une différence de pression et le courant partiel amenant la phase disposée au préalable est réglé par ajustement de la vitesse périphérique du rotor.

12. Utilisation d'un homogénéisateur du type rotor/stator qui comporte un raccord supplémentaire par lequel une substance à incorporer de façon homogène peut être amenée directement sur le rotor et la substance n'entre en contact avec la phase aqueuse externe que dans les couronnes dentées de l'homogénéisateur, pour la dissolution d'alkyléthersulfates dans de l'eau ou pour la production de compositions contenant des alkyléthersulfates.
